# EUROPEAN PATENT APPLICATION

(11) **EP 1 627 910 A1**
(43) Date of publication of application: **22.02.2006**
(21) Application number: 04733153.3
(22) Date of filing: 14.05.2004
(51) Int. Cl.: C12M 3/00, A01H 4/00, C12N 5/04, C12N 1/14, C12N 1/20, C12Q 1/04

(54) **APPARATUS FOR CULTURING ORGANISM AND CULTURE METHOD**

(30) Priority: 15.05.2003 JP 2003137812
(71) Applicant: Phytoculture Control Co., Ltd.,, Izumisano-shi, Osaka 598-0022 (JP)
(72) Inventor: Hasegawa, Ryou, Izumisano-shi, Osaka 598-0022 (JP); Suzumura, Daisuke, Izumisano-shi, Osaka 598-0022 (JP); Kimura, Takuji, Izumisano-shi, Osaka 598-0022 (JP)
(74) Representative: von Kreisler, Alek
(86) International application number: PCT/JP2004/006870
(87) International publication number: WO 2004/101736

(57) **Abstract**

An organism-culture apparatus, which can be conveniently prepared, can be stored for a long period, can be efficiently handled, and can be adapted to various organisms and culture conditions without restricting a kind of an organism to be cultured or the culture condition, and can be recycled after sterilization and washing is provides. The organism-culture apparatus, which comprises a microporous body retaining a culture medium, wherein an organism is cultured on a surface of the microporous body.

## Description

### FIELD OF THE INVENTION

The present invention relates to an apparatus and a method for culturing an organism. More particularly, the present invention relates to an apparatus and a method for culturing an organism, for the purpose of an examination of a microorganism present in a food and the like by the culture. Moreover, the present invention relates to an apparatus and a method for culturing an organism which can conveniently and efficiently culture the organism for a long period.

### BACKGROUND OF THE INVENTION

In a field of a food examination, or a sanitary examination where a medical or sanitary product is handled, an examination is generally conducted by plating a micro-organism collected from a sample onto an agar medium or by mixing a suspension of a collected micro-organism with a melted agar followed by an incubation of the medium. Thereafter, emerged colonies are counted visually or by a stereomicroscope.

As an apparatus aiming at such the micro-organism culture, there are disclosed a micro-organism-culture apparatus and medium having a water-retaining ability (see WO 97/024432).

However, the medium disclosed in WO 97/024432 relates to a dried medium. Although the medium is converted into one where a micro-organism can grow by adding water thereto, there is clearly defmed no amount of water to be added. Therefore, the medium may have a medium concentration not suitable for a micro-organism growth when an amount of water varies.

In addition, there is described in WO 97/024432 that the number of micro-organism colonies can be correctly counted even though the micro-organism infiltrates into a porous matrix layer, since a highly viscous water-soluble polymer is dissolved out with water contained in a test sample to expand in the porous matrix layer and, thereby, the micro-organism is pushed out to a surface of the porous matrix layer due to the highly viscous water-soluble polymer. But, there is a high possibility that a correct counting of colonies can not be conducted, because, in general, it is believed that a cavity in the porous matrix has not a simple structure, and at least a part of the micro-organism is captured in the cavity, and remains therein, without being pushed out with the water-soluble polymer.

In addition, there is disclosed a porous carbon material for the culture of an organism made of a porous carbon raw material (see JPA 188574/1997).

However, there is described in JPA 188574/1997 that the material has 5-50 µm of cavity diameter. In addition, there is described that the micro-organism has 1-2 µm of size and a cavity is required to have a diameter five-times the size of the micro-organism. Accordingly, in view of such description, it is believed that the material is an apparatus for the culture of the micro-organism in the cavity. However, in the case where the micro-organism is grown in the cavity, the colonies can be visually counted when the material is transparent, but it can not when the material is opaque. Accordingly, it is unsuitable as a culture apparatus aiming at the examination. In addition, in the case of a culture of a plant cell or a plant body, a plant tissue such as a root may infiltrates into a pore when the pore has such a size and, as the result, there is a high risk that the plant is damaged upon handling such as repotting.

Hitherto, a solid medium such as an agar medium has been utilized in a general examination culture. Accordingly, it is necessary that the medium is melted and, dispersed into a container such as a petri dish to solidify before use. In addition, the prepared medium can not be stored for a long period, because moisture in the medium is easily evaporated after preparation. Accordingly, the prepared medium should be used within a relatively short period. In addition, in an examination work front, a work for preparing a medium is very inefficient and, thereby, it is substantially impossible. In addition, in the case where the micro-organism growing under an acidic pH condition such as lactic acid bacteria is to be examined, the agar medium does not solidify at such an acidic pH range, and an agar plate can not be prepared. In addition, a differentiation of an adventitious bud is often inhibited due to an agar contained in the medium depending on a kind of the organism to be cultured, such as a case of an anther culture of tobacco plant *(Nicotiana tabacum).* In order to culture such organism in the conventional agar medium, a highly-purified or specially-treated agar was necessary. In addition, the agar medium or the liquid medium placed in a container such as the petri dish has been discarded after sterilizing a cultured micro-organism, but this is disadvantageous in view of an environmental pollution and an economical cost.

Furthermore, in the future, a culture or growth of an organism such as micro-organisms and plants under an agravic condition (experiments during an interplanetary travel or in a space station) is expected. In such the case, the culture on the agar medium requiring melting or heating of the agar, or potting requiring soil is believed to be difficult in view of a safety, an oxygen consumption, and an increased weight accompanying an increased cost. In addition, a use of a liquid medium requiring agitation or stirring is also believed to be difficult and disadvantageous for an organism culture or growth, because a liquid floats to form a globular shape under such the condition.

Then, there has been a need for a culture apparatus used for an examination of micro-organism which can solve problems as described above. In addition, there has been a need for an organism-culture apparatus which can culture micro-organisms and plants for a long period in addition to a short period, using the same apparatus.

### DISCLOSURE OF THE INVENTION

The present inventors studied intensively in view of the aforementioned problems and, as a result, found that the problems can be solved by making a particular microporous body retain the culture medium by the capillary action thereof, culturing an organism on a surface of the microporous body, and optionally supplying the microporous body with the medium to continue the culture, which resulted in completion of the present invention.

That is, in the first aspect, the present invention provides an organism-culture apparatus, which comprises a microporous body retaining a culture medium, wherein an organism is cultured on a surface of the microporous body.

According to the first aspect of the present invention, there can be provided an organism-culture apparatus, which can be conveniently prepared, can be stored for a long period, can be adopted without being restricted by a temperature and a pressure, a kind and a pH of the culture medium to be used, and a kind of an organism, and can be recycled after sterilization and washing. In addition, the organisms to be cultured can be correctly examined such as by counting colonies of the micro-organism on a surface of the microporous body, or cultured organisms such as plants can be separated in an intact state, because the organism is propagated or grown on the surface of the microporous body. Furthermore, in the culture or growth of the organism such as micro-organisms and plants in space, the apparatus of the present invention has a high safety, can suppress an oxygen consumption upon preparation and an increased cost accompanied with an increased weight, and can suitably culture and propagate the organism without releasing the culture medium from the microporous body.

In addition, in the organism-culture apparatus of the present invention, the microporous body can retain preferably 5-300% (wt/wt), more preferably 7-250% (wt/wt), and most preferably 8-200% (wt/wt) of the culture medium or water based on the weight thereof. Thereby, in addition to advantages as described above, an amount of the culture medium retained by the microporous body can be properly adjusted depending on a kind of the organism to be cultured, a culture duration, and a utility after the culture. In addition, an effluence of the micro-organisms or the like placed on the surface of the microporous body due to leaving of the culture medium on the surface of the microporous body and an increase in a weight of the apparatus upon a carriage to a space can be further suppressed. When an amount of the culture medium which can be retained by the microporous body is below 5%, there is a possibility that the organism to be cultured can not utilize a sufficient amount of the culture medium, being not preferable.

In addition, in the organism-culture apparatus of the present invention, the microporous body retains an amount of the culture medium retained by the capillary action thereof. Thereby, in addition to the advantages as described above, the organisms can be cultured while the microporous body retains only an amount of the culture medium which is required and sufficient for the organism culture, and an effluence of the organism to be cultured from the surface of the microporous body can be prevented.

In addition, preferably, the organism-culture apparatus of the present invention does not comprise soil. Thereby, in addition to the advantages as described above, an increased weight due to soil in the case of a plant culture can be suppressed and the grown plant can be separated from the organism-culture apparatus without damaging a root thereof or the like. In addition, chemically changeable factors such as a buffering or ion-exchanging ability of soil can be eliminated or controlled.

In addition, in the organism-culture apparatus of the present invention, the microporous body has a cavity diameter of preferably 5 µm or smaller, more preferably 3 µm or smaller, yet more preferably 2 µm or smaller, and most preferably 1 µm or smaller. In addition, cavities having a diameter of 1 µm or smaller are present in the microporous body in a distribution ratio of preferably 70% or larger, more preferably 85% or larger, yet more preferably 90% or larger, and most preferably 95% or larger based on a total volume of cavities. Thereby, in addition to the advantages as described above, the micro-organism and the like can be exactly counted because the organism such as the micro-organism is propagated or grown only on a surface of the microporous body without infiltrating into an interior of the microporous body and, in the growth of the plant, the plant can be separated from the microporous body in an intact state, without damaging a lodged root thereof or the like. When the cavity diameter exceeds 5 µm, the colony can not be exactly counted because the micro-organism may infiltrate into the cavity, and the plant may be damaged upon separation from the microporous body because a root hair may infiltrate into the cavity, being not preferable.

In addition, in the organism-culture apparatus of the present invention, a porosity of the microporous body is preferably 10-80% (vol/vol), more preferably 15-60% (vol/vol), and most preferably 18-50% (vol/vol). Thereby, in addition to advantages as described above, an amount of the culture medium retained by the microporous body can be controlled, and a weight of the microporous body itself can be suppressed. When the porosity is smaller than 10% , an amount of the culture medium suitable for the culture of the organisms can not be retained. On the other hand, when it exceeds 80% , a strength of the microporous body is lowered, being not preferable.

In addition, in the organism-culture apparatus of the present invention, preferably, the microporous body is a fired product of a non-metal inorganic solid material. Thereby, in addition to the advantages as described above, there can be provided the organism-culture apparatus having an excellent moldability, a light weight, and an excellent durability. In addition, when the microporous body of the organism-culture apparatus of the present invention is the fired product of the non-metal inorganic solid material, it retains preferably 5-50% (wt/wt), more preferably 7-25% (wt/wt), and most preferably 8-20% (wt/wt) of water or the culture medium, and it has a porosity of preferably 10-50% (vol/vol), more preferably 15-40% (vol/vol), and most preferably 18-37% (vol/vol).

In addition, in the organism-culture apparatus of the present invention, preferably, the microporous body is an open-cell type plastic foam. Thereby, in addition to the advantages as described above, the organism-culture apparatus of the present invention can be adapted to a variety of utilities, because the microporous body have an excellent moldability, a variety of shapes and a light weight. In addition, when the microporous body of the organism-culture apparatus of the present invention is the open-cell type plastic foam, it retains preferably 10-300% (wt/wt), more preferably 20-250% (wt/wt), and most preferably 30-200% (wt/wt) of water or the medium, and it has a porosity of preferably 10-80%(vol/vol), more preferably 15-60% (vol/vol), and most preferably 18-50% (vol/vol).

In addition, because the fired product of a non-metal inorganic solid material and the open-cell type plastic foam can be molded in thin, the organism-culture apparatus of the present invention can be formed so as to have a thin shape and, thereby, it can be suitably adapted to the culture or growth of the organism in the space station or the like where a culture area or a weight is restricted.

In addition, the organism-culture apparatus of the present invention is sealed in a sterile condition. Thereby, in addition to the advantages as described above, the organism-culture apparatus can be stored for a long period, and it can be conveniently used only by taking it out from a sealed condition under a usual environment or a sterile condition. For example, the organism-culture apparatus of the present invention may be conveniently adapted to the micro-organism culture by storing the microporous body, in which the culture medium has been retained in advance, in an aseptic condition with a conventional sealing means such as a retort pouch, and taking it out on a spot.

In addition, in the organism-culture apparatus of the present invention, preferably, the organism to be cultured is a micro-organism. Thereby, in addition to the advantages as described above, a desired micro-organism can be cultured and, thereafter, the presence thereof can be examined, or the micro-organism can be isolated.

In addition, in the organism-culture apparatus of the present invention, preferably, the micro-organism is bacteria, yeast, or fungi. Thereby, in addition to the advantages as described above, a particular micro-organism can be examined.

In addition, the organism-culture apparatus of the present invention is, preferably, for a food examination. Thereby, in addition to the advantages as described above, the micro-organism or the like which may be harmful to an animal such as a human that takes the food can be qualitatively or quantitatively examined. Alternatively, the presence of a useful micro-organism in the food can be examined, or the micro-organism can be isolated.

In addition, in the organism-culture apparatus of the present invention, preferably, the organism to be cultured is plants. Thereby, in addition to the advantages as described above, the desired plant can be cultured or grown and, thereafter, optionally, a treatment such as dedifferentiation, redifferentiation, transformation and the like may be carried out thereon.

In addition, in the second aspect, the present invention provides an organism-culture apparatus comprising a microporous body, wherein water in a culture medium retained in the microporous body has been substantially removed by drying, and wherein an organism is cultured on a surface of the microporous body to which an amount of water which has been removed, that is, an amount of water retainable by the microporous body, is added before use to restore the culture medium.

According to the second aspect of the present invention, in addition to the advantages of the first aspect, the apparatus can be stored for a longer period, it can be transported in its light weight (dried) state, and it can be conveniently used only by adding a predetermined amount of water.

In addition, in the third aspect, the present invention provides an organism-culture apparatus, which comprises one or more of microporous bodies retaining a culture medium and a holding means which sealably holds the microporous bodies, wherein an organism is cultured on a surface of the microporous body.

According to the third aspect of the present invention, in addition to advantages of the first and second aspects, plurality kinds of organisms can be cultured or grown on a plurality of microporous bodies, each retaining the same or different kind of the culture medium. In addition, one or more kinds of organisms can be cultured or grown under multiple conditions. In addition, the organism-culture apparatus can be formed into a portable type suitable for a collection of the organism in an outdoor environment.

In addition, in the fourth aspect, the present invention provides a method of examining a micro-organism, which comprises steps of:
(1) sterilizing a microporous body which has retained a culture medium by the capillary action thereof, or allowing a pre-sterilized microporous body to retain a pre-sterilized culture medium under a sterile condition;
(2) contacting a sample with the microporous body; and
(3) counting colonies formed on a surface of the microporous body after a culture of the microporous body under a given condition for a given period.

According to the fourth aspect of the present invention, there can be provided a method of examining, which can be conveniently prepared and which can be adapted without being restricted by temperature and a pressure, a kind and a pH of the culture medium to be used, and a kind of a micro-organism, and can be recycled after sterilization and washing. In addition, the micro-organisms to be cultured can be correctly examined such as by counting colonies of the micro-organism on a surface of the microporous body, because the micro-organism is propagated or grown on the surface of the microporous body. Furthermore, in the examination of the micro-organisms in a space station, the examination can be carried out in a high safety, and an oxygen consumption upon preparation and an increased cost accompanied with an increased weight can be suppressed, and it can suitably culture the micro-organism without releasing the culture medium from the microporous body.

In addition, in the method of examining of the present invention, preferably, the culture in the step (3) is carried out at -50 to 300 °C for shorter than 6 months. Thereby, the micro-organism present in the sample can be examined within a short period.

In addition, in the method of examining of the present invention, preferably, the micro-organism to be examined is bacteria, yeast or fungi. Thereby, the micro-organism which propagates and forms colonies by utilizing the culture medium can be examined.

In addition, in the method of examining of the present invention, preferably, the micro-organism present in the food is examined. Thereby, the micro-organism, which may be harmful to an animal such as a human that takes the food, can be qualitatively and quantitatively examined. Alternatively, a presence of a useful micro-organism in the food can be examined.

In addition, in the fifth aspect, the present invention provides a method of culturing an organism, which comprises steps of:
(1) sterilizing a microporous body which has retained a culture medium by the capillary action thereof, or allowing a pre-sterilized microporous body to retain a pre-sterilized culture medium under a sterile condition;
(2) adhering the organism to a surface of the microporous body; and
(3) after a culture of the microporous body under a given condition for a given period, successively supplying the microporous body with the culture medium such that an amount of the culture medium retained by the microporous body becomes an amount of the culture medium retained by the capillary action of the microporous body.

According to the fifth aspect of the present invention, the organism can be cultured by allowing the microporous body to retain only an amount of the culture medium which is required and sufficient for the organism culture.

In addition, in the method of culturing an organism of the present invention, preferably, the organism to be cultured is micro-organisms. Thereby, in addition to the advantages of the fifth aspect, a desired micro-organism can be cultured and, thereafter, the cultured micro-organism can be isolated or examined for a presence thereof.

In addition, in the method of culturing an organism of the present invention, preferably, the micro-organism is bacteria, yeast or fungi. Thereby, in addition to the advantages as described above, a particular micro-organism can be examined.

In addition; in the method of culturing an organism of the present invention, preferably, the organism to be cultured is plants. Thereby, in addition to the advantages as described above, a desired plant cell or tissue can be cultured or a desired plant body can be grown and, optionally, a treatment such as dedifferentiation, redifferentiation, transformation and the like may be carried out thereon.

The term "organism" used herein generally includes a tissue or a cell derived from micro-organisms, fungi, plants and animals. Then, the term used herein "micro-organism" includes bacteria, yeast and fungi, and the term "plant" used herein includes cells and tissues of plants as well as plant bodies. The cell and tissue of plants include one or more of cells and plant callus, and the plant body includes non-germinated seeds, germinated seedlings, plants at various growth stages, and parts of plant such as a leaf piece and an anther separated form such plants.

In addition, the term "cavity" used herein means all communicating pores into which water or the culture medium can infiltrate by immersing the microporous body of the organism-culture apparatus into them. The term "cavity diameter" used herein means a diameter of such the pore, and the term "porosity" means a ratio of a volume occupied by such the pore to a volume of the microporous body.

### BRIEF DESCRIPTION OF THE DRAWINGS

Figure 1 is a perspective view showing one aspect of the organism-culture apparatus of the present invention.

Figure 2 is a perspective view showing another aspect of the organism-culture apparatus of the present invention.

Figure 3 is a perspective view showing another aspect of the organism-culture apparatus of the present invention.

Figure 4 is a perspective view showing another aspect of the organism-culture apparatus of the present invention.

Figure 5 is a photograph substituted for a drawing showing a leaf piece of *Arabidopsis thaliana* colombia on 0 day of the culture using the organism-culture apparatus of the present invention.

Figure 6 is a photograph substituted for a drawing showing a leaf piece of *Arabidopsis thaliana* colombia after 10 days of the culture using the organism-culture apparatus of the present invention.

Figure 7 is a photograph substituted for a drawing, showing a leaf piece of *Arabidopsis thaliana* colombia after 21 days of the culture using the organism-culture apparatus of the present invention.

Figure 8 is a photograph substituted for a drawing, showing a flora of fungi *(Penicillium chrysogenum)* after 4 days of the culture using the organism-culture apparatus of the present invention.

Figure 9 is a photograph substituted for a drawing, showing a flora of fungi (*Penicillium chrysogenum*) after 10 days of the culture using the organism-culture apparatus of the present invention.

Figure 10 is a photograph substituted for a drawing, showing a flora of fungi (*Penicillium chrysogenum*) after 13 days of the culture using the organism-culture apparatus of the present invention.

Figure 11 is a photograph substituted for a drawing, showing a flora of fungi *(Penicillium chrysogenum)* after 0 day of the culture using the organism-culture apparatus of the present invention.

Figure 12 is a photograph substituted for a drawing, showing a flora of fungi *(Penicillium chrysogenum)* after 13 day of the culture using the organism-culture apparatus of the present invention.

Figure 13 is a photograph substituted for a drawing, showing a flora of fungi *(Penicillium chrysogenum)* after 21 day of the culture using the organism-culture apparatus of the present invention.

Figure 14 is a photograph substituted for a drawing, showing a flora of fungi *(Penicillium chrysogenum)* after 24 day of the culture using the organism-culture apparatus of the present invention.

Figure 15 is a photograph substituted for a drawing, showing a flora of bacterium *(Bacillus subtilis)* after 6 days of the culture using the organism-culture apparatus of the present invention.

Figure 16 is a photograph substituted for a drawing, showing a flora of bacterium *(Bacillus subtilis)* after 13 days of the culture using the organism-culture apparatus of the present invention.

### DETAILED DESCRIPTION OF THE PREFERRED EMBODIMENTS

Next, embodiments of the organism-culture apparatus of the present invention will be described with referring to drawings.

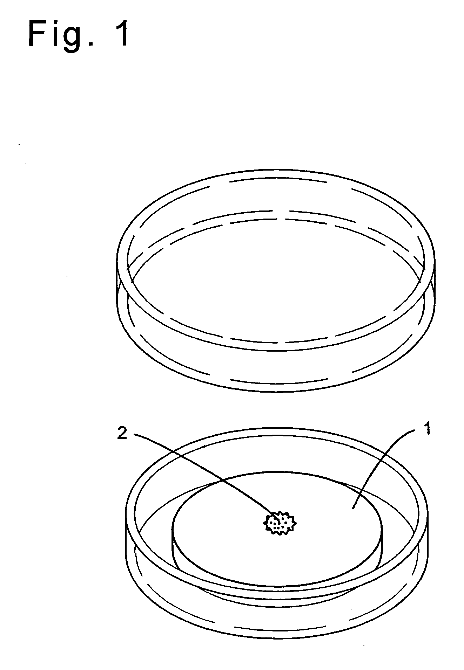

Firstly, the first embodiment of the organism-culture apparatus of the present invention is an organism-culture apparatus comprising a microporous body (1) retaining a culture medium as illustrated in Figure 1. When an organism to be cultured (2) is placed on a surface of the microporous body (1), it can propagate, dedifferentiate, redifferentiate, differentiate, or grow by absorbing the culture medium retained in the microporous body (1).

The culture medium to be used in the organism-culture apparatus of the present invention is not particularly limited, but any culture media may be used as far as they can be retained by the microporous body and can propagate, dedifferentiate, differentiate, regenerate, store, select, separate, crossbreed, or grow the desired organism, to which a variety of amino acids, vitamins, enzymes, antibiotics, osmoregulatories, buffering agents, natural materials (such as yeast extract), antifreezing agants and the like may be added depending upon a purpose. Examples thereof include, for example, the culture media for bacteria such as a potato sucrose medium, a BL medium, a CW medium, a modified CCFA medium, a B-CYE alpha medium, a WYO alpha medium, a DNase medium, a PS latex medium, a TCBS medium, a BGLB medium, an EC medium, a CVT agar, an EMB medium, a BCM O157 medium, an NAC agar, an OF medium base, a dextrose-phosphate-peptone medium, a Rusell medium, a Kligler medium, a TSI medium, a SIM medium, a Simmons sodium citrate medium, a malonate medium, a urea medium, a Christensen urea medium, a lysine iron agar medium, a medium for testing lysine decarbonization, an LIM medium, an OIML medium, a VPOF medium, an SS medium, an SS-SB medium, a MacConkey medium, a DHL medium, a brilliant green medium, an XLD medium, a Rappaport broth, a Hajna tetrathionate broth base, a selenite broth base, an SBG sulfur broth base, a tetrathionate broth, an EEM broth, a heart infusion medium, a brain heart infusion medium, an SCD medium, an SCDLP medium, a BTB lactose medium, a Drigalski medium, an SCDLP broth, a lactose broth JP medium, a cowpat for methanogenic bacteria, an MS-1 (1.5% casamino acid, 0.01% cysteine, 0.01% tryptophan, 0.05% sodium citrate, 0.2% sodium succinate, 0.05% K₂HPO₄, 0.05% KH₂PO₄, 30.01% KNO, 2.0% MgSO₄·7H₂O, 0.005% FeSO₄·7H₂O, 22-26% NaCl), an MS-2 medium (0.5% casamino acid, 1.0% yeast extract, 0.5% peptone, 0.3% sodium citrate, 0.5% KCl, 2.0% MgSO₄·7H₂O, 0.005% FeSO₄·7H₂O, 25% NaCl) and an MS-3 medium (1.0% yeast extract, 0.5% MgCl₂·6H₂O, 0.5% NH₄Cl, 25% NaCl) for high halophilic bacteria, an MSA-4 medium (1.0% peptone, 0.3% sodium citrate, 2.0% MgSO₄·7H₂O, 0.2% KCl, 5.0% NaCO₃·10H₂O, 25% NaCl) for alkaliphilic and high halophilic bacteria, a YSG medium for thermophilic and acidophilic bacteria, an MH-1 medium (1.0 g of yeast extract, 1.0 g of Tryptone, 30 g of NaCl, 3.5 g of MgSO₄·7H₂O, 2.8 g of MgCl₂·6H₂O, 0.2 g of FeSO₄· 7H₂O, 0.33 g of KCl, 0.2 g of NH₄Cl, 50 mg of NaBr, 20 mg of H₃BO₃, 0.5 g of KH₂PO₄, 7.5 mg of SrCl·6H₂O, 10 mg of (NH₄)₂SO_{4,}, 0.1 mg of Na₂WO₄·2H₂O, 50 mg of KI, 0.75 g of CaCl₂·2H₂O, 2 mg of NiCl₂·6H₂O, 1 mg of Resazurine, 10 ml of trace ingredients solution (1.5 g of nitriro triacetate, 3 g of MgSO₄·7H₂O, 0.5 g of MnSO₄·7H₂O, 1 g of NaCl, 0.18 g of ZnSO₄·7H₂O, 10 mg of CuSO₄· 5H₂O, 20 mg of KAl(SO₄)₂·7H₂O, 10 mg of H₃BO₃, 10 mg of Na₂MoO₂·2H₂O, 25 mg of NiCl₂·6H₂O, 0.3 mg of Na₂SeoO₃·5H₂O per 1L of distilled water), 25 g of Sulfer, 25 g of Na₂S·9H₂O per 1L of distilled water), an MH-2 medium (0.01% yeast extract, 0.01% casamino acid, 0.1% carbon source, 0.02% NaCl, 0.03% KH₂PO₄, 0.13% (NH₄)₂SO₄, 0.025% MgSO₄·7H₂O, 0.005% CaCl₂·2H₂O, glucose), an MH-3 medium (5 g of Bacto Peptone, 1 g of Bacto Yeast Extract, 0.1 g of FeC₅H₅O₇, 19.45 g of NaCl, 5.9 g of MgCl₂, 3.24 g of Na₂SO_{4,}, 1.8 g of CaCl₂, 0.55 g of KCl, 0.16 g of NaHCO₃, 0.08 g of KBr, 0.034 g of SrCl₂, 0.022 g of H₃BO₃, 0.004 g of sodium silicate, 0.0024 g of NaF, 0.0016 g of NH₄NO₃, 0.008 g of Na₂HPO₄, 10 g of casein or starch per 1L of distilled water) for thermophilic archaebacteria and the like; a culture medium for fungi such as a modified Ohta medium, a Hamada's EBIOS-sucrose medium, an M medium, an MYP medium, a PDA medium, an Ohta medium, a Mozel b-medium, a Wessels and Niderpruem minimum medium for mating, a Kerruish and Da Costa medium, a Goodey and Lucetohole medium, a Czapek medium, an Yeast infusion medium, an Wickerham synthetic medium, an MY medium, an oatmeal medium, a modified Gorodkowa medium, a Christensen's urea medium, a Henneberg medium, a Czapek-Dox medium, a Uschinsky medium, a thioglycolate medium for anaerobic fungi, a Kleyn sodium acetate medium, an yeast complete synthetic medium (Wickerham), a succinate-nitrate medium, a Gorodkowa medium, a cornmeal medium, a nitrate medium, a Fowells sodium acetate medium, a Lindegren medium and the like, to which a variety of amino acids, vitamins, enzymes, antibiotics, osmoregulatories, buffering agents, natural materials (such as yeast extract), antifreezing agants and the like may be added depending upon a purpose; a culture medium for a plant tissue such as an MS (Murashige-Skoog) medium, a B5 medium, a W medium, an NT, medium, a Kao8P medium, an LS medium, an H medium, a KC medium, an HB medium, WPM, a Kassanis medium, a Neelsen's medium, a Galzy medium, a Nitsh and Nitsh medium, a Noushi medium and the like, to which a variety of plant hormones, amino acids, vitamins, antibiotics, osmoregulatories, buffering agents, natural materials (such as yeast extract), enzymes, antifreezing agents and the like may be added depending upon a purpose; a culture medium for growing the plants such as water, or a solution which contains an ingredient required for germinating and growing a plant seed such as an inorganic element such as nitrate nitrogen, an ammonia nitrate, phosphorus, potassium, calcium, magnesium, iron and manganese, copper, zinc, molybdenum, boron and the like, a variety of vitamins such as thiamine, pyridoxine, nicotinic acid, biotin, folic acid and the like, a natural material such as coconut milk, casein hydrolysate, yeast extract and the like, an organic nitrogen source such as glutamic acid, aspartic acid, alanine and the like, a plant growth regulating material such as auxin, cytokinin, gibberellin and the like, a carbon source such as dextrose, sucrose, fructose, maltose and the like, an antibiotic such as kanamycin, hygromycin and the like, an agrochemical such as basta, and the like.

Next, a microporous body (1) used in the organism-culture apparatus of the present invention can retain preferably 5-300% (wt/wt), more preferably 7-250% (wt/wt), and most preferably 8-200% (wt/wt) of the culture medium at 20 °C, and has ability to absorb water the same amount as that of the culture medium, although it may vary depending on a kind of the culture medium to be used. When the microporous body used in the organism-culture apparatus of the present invention is a fired product of a non-metal inorganic solid material as described below, it can retain preferably 5-50% (wt/wt), more preferably 7-25% (wt/wt), and most preferably 8-20% (wt/wt) of water or the culture medium. On the other hand, when the microporous body used in the organism-culture apparatus of the present invention is a plastic foam, it can retain preferably 10-300% (wt/wt), more preferably 20-250% (wt/wt), and most preferably 30-200% (wt/wt) of water or the culture medium. In addition, the microporous body used in the organism-culture apparatus of the present invention has a porosity of preferably 10-80% (vol/vol), more preferably 15-60% (vol/vol), and most preferably 18-50% (vol/vol). When the microporous body used in the organism-culture apparatus of the present invention is a fired product of a non-metal inorganic solid material, it has a porosity of preferably 10-50% (vol/vol), more preferably 15-40% (vol/vol), and most preferably 18-37% (vol/vol). On the other hand, when the microporous body is a plastic foam, it has a porosity of preferably 10-80% (vol/vol), more preferably 15-60% (vol/vol), and most preferably 18-50% (vol/vol). As described above, the microporous body contains a number of communicating pores, and can absorb and retain water by the capillary action thereof. Preferably, the microporous body retains the culture medium at the same amount of as that can be retained by the capillary action thereof. In order to allow the microporous body to retain the culture medium, the microporous body in a dry state is immersed in an adequate amount of the culture medium for several hours to several days and, thereafter, it is removed from the culture medium, and the culture medium attached to the surface of the microporous body is removed by wiping away and the like. Alternatively, a predetermined amount of the culture medium may be absorbed from a surface of a dried microporous body.

The microporous body contains pores having a cavity diameter of preferably 0.5 µm or smaller, more preferably 3 µm or smaller, still preferably 2µm or smaller and most preferably 1 µm or smaller. In addition, a cavity diameter distribution ratio of the pore having a cavity diameter of 1 µm or smaller in the microporous body is preferably 70% or larger, more preferably 85% or larger, still more preferably 90% or larger, and most preferably 95% or larger of a total pore volume. The cavity diameter distribution ratio of the pore having a cavity diameter of 0.3 µm or smaller in the microporous body is 30-50%. The cavity diameter distribution ratio of the pore having a cavity diameter of 0.3-0.5 µm in the microporous body is 10-20%. The cavity diameter distribution ratio of the pore having a cavity diameter of 0.5-1 µm in the microporous body is 20-40% , and that of the pore having a cavity diameter of 1-3 µm is 5-15%. In addition, the microporous body has a bulk density of usually 0.1-3.0 g/cm³, preferably 0.2-2.5 g/cm³, and most preferably 0.3-2.2 g/cm³. Specifically, when the microporous body is the fired product of the non-metal inorganic solid material as described below, it has a bulk density of preferably 1.5-3.0 g/cm³, more preferably 1.8-2.5 g/cm³, and most preferably 1.9-2.2 g/cm³. On the other hand, when the microporous body is the plastic foam as described below, it has a bulk density of preferably 0.1-1.5 g/cm³, more preferably 0.2-1.0 g/cm³, and most preferably 0.3-0.7 g/cm³.

These cavity diameter, porosity, cavity diameter distribution ratio and bulk density of the microporous body can be controlled by a raw material and a condition for manufacturing the microporous body as described below. In addition, the capillary action of the microporous body as described above can be adjusted to change an amount of the culture medium retained by the microporous body, by controlling the cavity diameter, porosity and cavity diameter distribution ratio in the condition for manufacturing the microporous body.

In addition, the microporous body may be any one having aforementioned characteristics, but preferably it is composed of a material resistant to a high temperature and high pressure sterilizing treatment such as a treatment with an autoclave, and resistant to a culture condition or a culture medium condition such as strong alkaline, strong acidic, high temperature, low temperature, high salt concentration, high pressure, decompression, organic solvent, radiation or gravity-applying conditions or the like. Examples of the material of the microporous body include, for example, a non-metal inorganic solid material obtaining by kneading, forming and firing non-metal inorganic solid raw materials such as No. 10 clay, porcelain No.2 clay (Shiroyama Cerapot Co., Ltd.), Murakami clay (produced in Niigata Prefecture in Japan) and PCTG No. clay (Tono-Gaishi Co., Ltd.) according to the conventional method, as well as open-cell type plastic foam materials such as polyvinyl alcohol foam, polyurethane foam, polystyrene foam, vinyl chloride resin foam, polyethylene foam, polypropylene foam, phenol resin foam, urea resin foam and the like. In particular, when the non-metal inorganic solid raw material is made into a porous body which easily absorbs and releases water, it is preferable that those raw materials are fired while containing, for example, petalite and alumina at 50-60% by weight. Generally, the petalite preferably contains 76.81% by weight of SiO₂, 16.96% by weight of Al₂O₃, 4.03% by weight of LiO₂, 0.26% by weight of K₂O and 1.94% by weight of inevitable impurities. In addition, non-metal inorganic solid raw materials may contain a powdery inorganic foam. Further, the microporous body used in the organism-culture apparatus of the present invention is composed of a non-metal inorganic material, a strength of which is not substantially reduced or the shape of which is not deformed even when it has absorbed water.

As a method of forming a non-metal inorganic solid raw material, there are forming methods which are known in the art such as slip casting forming, extruding forming, press forming and potter's wheel forming. In particular, from a viewpoint of large scale production and a reduction in the cost, extruding forming method is preferable. In addition, drying after forming can be carried out using the ordinary methods and conditions known in the art. Subsequent firing of a formed body is not particularly limited as far as it is carried out according to the ordinary conditions and methods. For example, oxidative firing by which a desired pore is easily obtained can be selected. A firing temperature is 1000 °C to 2000 °C, preferably 1100 °C to 1500 °C, more preferably 1150 °C to 1300 °C, and most preferably 1250 °C. When a temperature for firing the non-metal inorganic solid raw material is lower than 1000 °C, a sulfur component easily remains and, on the other hand, when the temperature is higher than 2000 °C, a desired culture medium retaining ability is not obtained.

On the other hand, as a method of molding a microporous body composed of an open-cell type plastic foam, for example, there are melt foaming molding, solid phase foaming molding, casting foaming molding and the like.

Principal steps in melt foaming molding comprise melting and kneading, molding of an unfoamed sheet, heat foaming or extrusion foaming, cooling, cutting and processing. In solid phase foaming molding, a polymer is foamed in the solid phase or in the state near the solid phase. In addition, in casting foaming molding, a liquid raw material (monomer or oligomer) is cast and foamed while reacting in the air. In order to foam an open-cell type plastic foam, a foaming agent is generally used.

In addition, the microporous body (1) can be formed into a plate, disc, pillar, cylindrical type or the like depending upon a purpose of the culture, but preferably the microporous body formed into the disc type is used, which can be easily handled, can efficiently culture the organism, and can be stored compact during the culture.

Upon contact with the microporous body (1), the culture medium is absorbed into the microporous body via communicating pores in the microporous body by the capillary action of communicating pores, retained in an interior thereof, and supplied to the organism (2) placed on the surface of the microporous body to induce propagation, dedifferentiation, differentiation, regeneration and the like of the organism.

Examples of the organism (2) to be cultured using the organism-culture apparatus of the present invention include, for example, bacteria such as photosynthetic bacteria *(Rhodospillum molischianum, Rhodopseudomonas acidophila, Rhodomicrobium vannielii, Chromatium vinosum, Thiocapsa roseopersicina, Thiopedia rosea, Chlorobium limicola, Chlorobium phaeovibrioides, Pelodictyon clathratiforme,* purple photosynthetic bacteria (*Ectothiorhodospira halophila*)), gliding bacteria (*Myxococcus fulvus, Myxococcus coralloides, Myxococcus stipitatus, Myxococcus xanthus*), sheathed bacteria (*Sphearotilus natans*), budding bacteria, bacteria having an appendage (*Hyphomonas neptunium, Gallionella ferruginea*), spirochetes (*Spirochaeta icterohaemorrhagiae, Spirochaeta pallida, Spirochaeta aurantia*), spirillum, spiral or twist bacteria, gram-negative bacteria, aerobic bacilli or cocci (*Pseudomonas fluorescens, Pseudomonas aeruginosa, Pseudomonas ovalis, Pseudomonas gluconicum, Xanthomonas oryzae, Gluconobacter oxydans*), nitrogen-fixing bacteria (*Azotobacter chroococcum, Rhizobium leguminosarum, Rhizobium tfifolii, Rhizobium meliloti, Rhizobium phaseoli, Rhizobium japonicum, Clostridium pasteurianum*) , Methylomonadaceae (*Methylomonas methanica*), acetic acid bacteria (*Acetobacter aceti*), facultative anaerobic bacilli (*Escherichia coil, Enterobacter aerogenes*), typhoid bacilli (*Salmonella typhi*), *Salmonella typhimurium, Salmonella enteritidis,* dysentery bacilli (*Shigella typhimurium*), *Serratia marcescescens, Proteus vulgaris, Vibrio cholerae, Vibrio parahaemolyticus*)), anaerobic bacteria (*Bacteroides succinogenes*), aerobic cocci or coccobacilli (*Staphylococcus aureus, Staphylococcus epidermidis, Staphylococcus saprophyticus, Streptococcus pyogenes, Streptococcus agalactiae*, *Streptococcus viridans, Streptococcus pneumoniae, Enterococcus faecalis, Enterococcus faecium, Enterococcus avium, Corynebacterium diphtheriae, Bacillus subtilis, Bacillus anthracis, Bacillus cereus*), anaerobic cocci (*Nesseria gonorrhoeae*), gram-negative lithotrophic bacteria (*Nitrosomonas europaea, Nitrosococcus oceani, Nitrobacter hamburgensis, Nitrobacter vulgaris, Nitrobacter winogradskyi, Thiobacillus thiooxydans*), gram-positive cocci (glutamic acid fermenting bacteria (*Micrococcus glutamicus*), *Staphylococcus aureus, Spreptococcus lactis, Streptococcus bovis, Streptococcus mutans, Leuconostoc mesenteroides, Leuconostoc lactis, Pediococcus cerevisiae, Pediococcus acidilactici, Pediococcus pentosaceus, Lactobacillus delbrueckii, Lactobacillus rimae, Sporolactobacillus inulinus, Bacillus coagulans, Bacillus subtilis, Bacillus polymyxa, Bacillus maercerans, Bacillus pycnoticus*, anthrax bacteria (*Bacillus anthracis*), butylic acid fermenting bacteria (*Clostridium butyrium*), acetone-butanol fermenting bacteria (*Clostridium acetobutylicum*), *Clostridium sporogenes, Clostridium botulinum, Clostridium pefringens,* tetanus bacilli (*Clostridium tetani*), sulfur reducing bacteria (*Desulfotomaculum rumimis*), spore sarcina (*Sporosarcina ureae*)), bacteria associated with mycobacteria (diphtheria (*Corynebacterium diphtheriae*), *Corynebacterium fascians, Corynebacterium rathayi, Corynebacterium sepedonicum, Corynebacterium insidiosum, Corynebacterium flaccumfaciens, Actinomyces bovis, Nocardia farcinica, Streptomyces griseus, Streptomyces rameus, Streptomyces venezuelae, Streptomyces omiyaensis, Streptomyces aureofaciens, Streptomyces avellaneus, Streptomyces lutianus),* thermophilic bacteria (*Aeropyrum pernix, Aquifex aeolicus, Archaeoglobus fulgidus, Bacillus thermoleovorans, Methanococcus jannaschii, Methanothermus fervidus, Pyrobaculum aerophilum, Pyrobaculum calidifontis, Pyrobaculum islandicum, Pyrobaculum oguniense, Pyrococcus furiosus, Pyrococcus horikoshii, Pyrococcus kodakaraensis, Pyrococcus shinkaj, Pyrolobus fumarii, Rhodothermus obamensis, Saccharopolyspora rectivirgula, Sulfolobus acidocaldarius, Sulfolobus shibatae, Sulfolobus shibatae, Sulfolobus solfataricus, Sulfolobus tokodaii, Thermoactinomyces vulgaris, Thermococcus celer, Thermococcus kodakaraensis, Thermococcus litoralis, Thermococcus profundus, Thermococcus strain, Thermoplasma acidophilum, Thermoplasma volcanium, Thermotoga maritima, Thermotoga neapolitana, Thermus thermophilus),* methane bacteria *(Methanobacterium formicicum, Methanobacterium thermoautotrophicum, Methanobrevibacter arboriphilus, Methanobrevibacter ruminantium, Methanobrevibacter smithii, Methanococcus jannaschii, Methanoculleus chikugoensis, Methanopyrus kandleri, Methanosaeta concilii, Methanosarcina barkeri, Methanosarcina mazeii, Methanosphaera stadmaniae, Methanothermobacter thermautotrophicus),* halophilic bacteria (*Haloarcula japonica, Haloarcula marismortui, Halobacterium halobium, Halobacterium salinarium, Haloferax mediterranei, Haloferax volcanii, Halomonas variabilis, Natronobacterium pharaonis, Tetragenococcus halohila, Vibrio parahaemolyticus, Vibrio vulnificus*), cryophilic bacteria (*Colwellia psychrerythraea, Moritella marina, Yersinia enterocolitica, Yersinia pseudotuberculosis, Shewanella benthica),* barophilic bacteria (*Moritella japonica, Moritella yayanosii, Photobacterium profundum, Shewanella benthica, Shewanella violacea, Shewanella oneidensis),* acidophilic bacteria (*Aeropyrum pernix, Sulfolobus solfataricus, Sulfolobus tokodaii, Sulfolobus acidocaldarius, Thermoplasma acidophilum, Alicyclobacillus acidocaldarius, Alicyclobacillus acidoterrestris, Alicyclobacillus cycloheptanicus, Thiobacillus acidophilus, Acidianus brierleyi*), alkaliphilic bacteria (*Bacillus alcalophilus, Bacillus halodurans, Bacillus pasturii, Exiguobacterium aurantiacum*), radiation-resistant bacteria (*Deinococcus radiodurans, Micrococcus radiodurans, Bacillus cereus),* petroleum catabolizing bacteria HD-1 strain which was isolated at an oil field in Shizuoka Prefecture (CO₂-fixing type petroleum synthesizing or degrading bacteria), TK-122 strain, and organic solvent-resistant bacteria *(Pseudomonas putida* IH-2000 strain) and the like; micro-organisms such as a mycelium or a cell of filamentous fungus such as *Mucor, Rhizopus, Absidia, Phycomyces, Aspergillus* such as *Aspergillus niger, Aspergillus oryzae and Aspergillus tamarii* and the like, *Penicillium, Fusarium, Trichoderma, Monilia* as well as yeast *(Saccharomyces cerevisiae)* and the like; fungi such as enoki mushroom *(Flammlina velutipes),* shiitake mushroom *(Lentinula edodes),* bunashimeji mushroom *(Hypsizygus marmoreus),* fried chicken mushroom *(Lyophyllum decastes),* nameko mushroom *(Pholiota nameko),* inky cap *(Coprinus atramentarius),* sulphur tuft *(Naematoloma sublateritium),* puffball *(Lycoperdon gemmatum),* mannentake muchroom *(Ganoderma lucidum),* suehirotake muchroom *(Schizophyllum commune),* oyster mushroom *(Pleurotus ostreatus),* maitake mushroom *(Grifola frondosa),* matsutake mushroom *(Tricholoma matsutake),* yanagimatsutake mushroom *(Agrocybe cylindracea),* turkey tails *(Coriolus versicolor*) *,* brown yellow boletus (*Suillus luteus*)*,* larch boletus (*Suillus grevillei*)*,* amihanaiguchi (*Boletinus cavipes*)*,* honshimeji mushroom (*Lyophyllum shimeji*) and the like; the plant tissue or cell such as of a seed, a leaf, a shoot apex, a stem, a root, an anther, a filament, a growing point (a terminal bud, a lateral bud, a shoot apex, a root apex), an axillary bud, a scale, an ovary, an ovule, an embryo, a pollen, an adventitious bud, an adventive embryo and an adventitious root of useful plants such as bishop's flower (*Ammi majus*), onion (*Allium cepa*), garlic (*Allium sativum*), celery (*Apium graveolens*), asparagus (*Asparagus officinalis*), sugar beet (*Beta vulgaris*), cauliflower (*Brassica oleracea* var. *botrytis*), brusseles sprout (*Brassica oleracea* var. *gemmifera*), cabbage (*Brassica oleracea* var. *capitata*), rape (*Brassica napus*)*,* caraway (*Carum carvi*), chrysanthemum (*Chrysanthemum morifolium*), spotted hemlock (*Conium maculatum*)*,* coptis Rhizome (*Coptis japonica*)*,* chicory (*Cichorium intybus*), summer squash (*Curcurbita pepo*)*,* thorn apple (*Datura meteloides),* carrot (*Daucus carota*), carnation (*Dianthus caryophyllus*)*,* buckwheat (*Fagopyrum esculentum),* fennel *(Foeniculum vulgare),* strawberry (*Fragaria chiloensis),* soybean *(Glycine max*), hyacinth *(Hyacinthus orientalis),* sweet potato *(Ipomoea batatas),* lettuce (*Lactuca sativa*), birds-foot trefoil *(Lotus corniculatus, Lotus japonicus),* tomato *(Lycopersicon esculentum),* alfalfa *(Medicago sativa*), tobacco *(Nicotiana tabacum),* rice (*Oryza sativa*), parsley *(Petroselinum hortense),* pea *(Pisum sativum),* rose (*Rosa hybrida*), egg plant (*Solanum melongena),* potato (*Solanum tuberosum),* wheat (*Triticum aestivum*), maize (*Zea mays)* and the like; foliage plants such as snapdragon *(Antirrhinum majus*), mouse-ear cress *(Arabidopsis thaliana*)*,* croton (*Codiaeum variegatum),* cyclamen (*Cyclamen persicum),* poinsettia (*Euphorbia pulcherrima*), barberton daisy (*Gerbera jamesonii*), sunflower (*Helianthus annuus*), fish geranium (*Pelargonium hortorum*), petunia (*Petunia hybrida*), African violet (*Saintpaulia ionatha*), dandelion (*Taraxacum officinale*), torenia (*Torenia fournieri*), Dutch clover (*Trifolium repens*), cymbidium (*Cymbidium*) and the like; useful trees such as beat tree *(Azadirachta indica*), orange (*Citrus*)*,* common coffee (*Coffea arabica*), ribbon gum *(Eucalyptus),* para rubber tree (*Hevea brasiliensis),* holly *(Ilex aquifolium*), trifoliate orange *(Poncirus trifoliata*), almond *(Prunus amygdalus*), carolina poplar *(Populus canadensis),* oriental arborvitae (*Biota orientalis),* Japanese ceder *(Cryptomeria japonica),* Norway spruce (*Picea abies*)*,* pine genus (*Pinus*)*,* grapevine (*Vitis vinifera*)*,* apple (*Malus pumila*)*,* apricot (*Prunus armeniaca*)*,* persimmon (*Diospyros kaki*)*,* fig (*Ficus carica*)*,* chestnut (*Castanea crenata*) and the like.

The organism-culture apparatus of the present invention is applicable to a variety of fields including a step of the culture of the organism as described above, and for example, is applicable to the culture of the organism used in a food examination, a water examination, a soil examination, separation of the micro-organism from nature, a production of anticeptic seedling, a food poisoning examination, a bacterial examination of an apparatus or a machine, a virus examination of an aquatic product, a virus examination of a farm animal product, a virus examination of an agricultural farm product, an examination of drinking water, an epidemiologic study, a food production, an oxygen production, an amino acids, vitamins or enzymes production, an antibiotic production and the like.

In view of the effects of the present invention, the organism-culture apparatus of the present invention is preferably handled under an aseptic condition, and preferably is stored with aseptically sealing.

In addition, in another aspect, the organism-culture apparatus of the present invention can be restored by removing water from the microporous body retaining the culture medium once as described above and, thereafter, adding an amount of water which corresponds to the amount removed before the organism culture. Removal of water from the microporous body can be carried out by an ordinary procedure such as heating, decompression, freeze-drying and the like. Herein, the phrase "substantially removing water of the culture medium" refers to a state in which most of water in the culture medium, preferably 70-100% of water based on a weight of the culture medium retained in the microporous body, is removed by heating the microporous body retaining the culture medium, or by subjecting the microporous body to a reduced pressure condition, preferably to a freeze-drying.

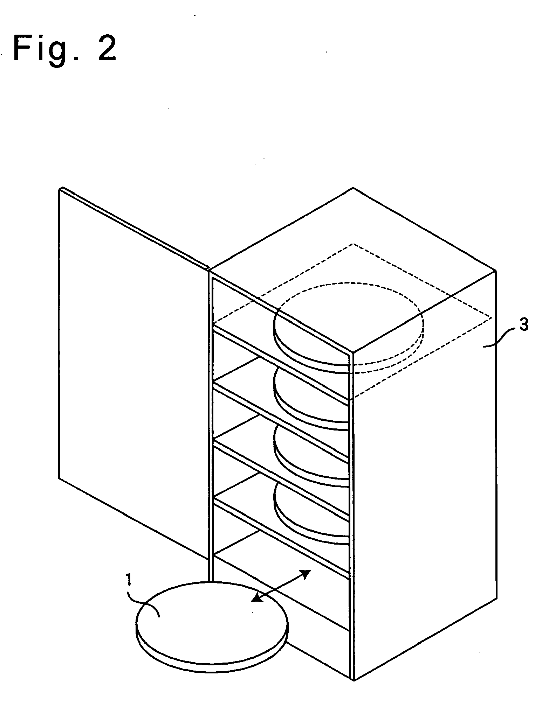

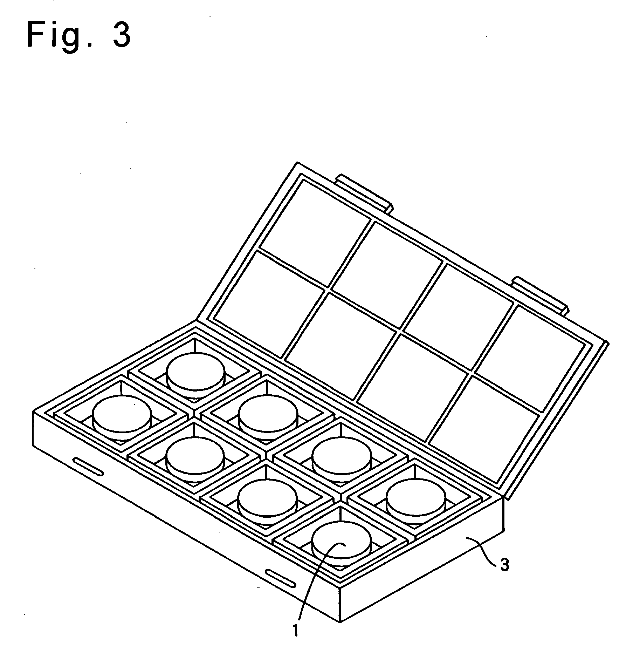

Furthermore, in still another aspect, the organism-culture apparatus of the present invention may be one comprising one or more of microporous bodies as described above retaining the culture medium, and a holing means for sealably holding the microporous bodies. Examples of the organism-culture apparatus in this aspect include a rack-type organism-culture apparatus as shown in Figure 2, which comprises a plurality of disc-type microporous bodies (1) retaining the culture medium, and a holding means (3) which can sealably hold the microporous bodies. This rack-type organism-culture apparatus can effectively utilize a space, since it can lamellarly hold the microporous bodies (1) before use or during the culture. In addition, the organism-culture apparatus of the present invention may be a palette-type organism-culture apparatus as shown in Figure 3, which can sealably hold a plurality of disc-type microporous bodies (1) retaining the culture medium. In this palette-type organism-culture apparatus, the microporous bodies (1) are detachable, and can be independently housed in each section when a lid is closed. Thereby, a plurality kind of organisms may be cultured, or one kind of the organism may be cultured under various conditions where, for example, a culture medium composition is changed, while a comparison is carried out between them.

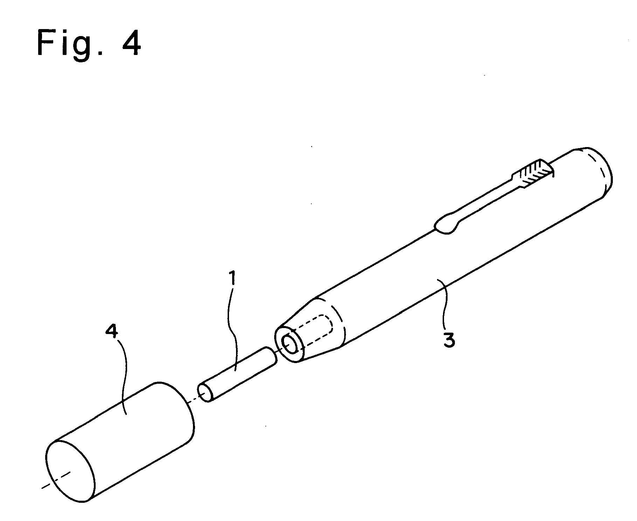

Furthermore, the organism-culture apparatus of the present invention may be a potable pen-type organism-culture apparatus as shown in Figure 4, in which the microporous body is sealed with a cap (4). This potable pen-type organism-culture apparatus is suitable for use in an outdoor collection of an organism. The organism can be collected by removing the cap upon the collection, and contacting an end portion of the microporous body retaining the culture medium with a desired sample. Thereafter, the cap can be closed again, and the potable pen-type organism culturing apparatus is brought to a laboratory. Then, the cap can be removed, and the end portion of the microporous body can be streaked on a larger scale medium to transfer a collected organism to culture thereon. In the organism-culture apparatus in this aspect, the microporous body (1) is detachable, and it may be detached after the collection and a fresh microporous body may be mounted.

Also, the present invention provides a method of qualitatively and quantitatively examining a micro-organism using the aforementioned organism-culture apparatus. Firstly, the microporous body which has been allowed to retain the culture medium by the capillary action thereof is sterilized, or a pre-sterilized microporous body is allowed to retain a pre-sterilized culture medium under a sterile condition. Then, the desired sample, that is, a material in which the micro-organism may be present such as a food, river water, seawater, soil, an apparatus, a machine, drinking water, an aquatic product, a farm animal product, an agricultural farm product, a human body sample as well as an animal and plant sample is directly contacted with the microporous body, or the organism such as the micro-organism separated from the material is indirectly adhered to the microporous body using a cotton bud or a platinum needle. Alternatively, a given amount of suspension prepared by suspending the organism attached to the cotton bud or the platinum needle in water may be plated on the microporous body. Thereafter, the microporous body on which the desired organism is attached is cultured under a given condition suitable for the organism for a given period, and a colony or a cell agglomerate formed on the surface of the microporous body may be observed or counted visually or under a stereomicroscope. Optionally, various dyes may be used to stain the organism upon observation or counting.

In addition, in the method of examining of the present invention, a culture period is shorter than six months, preferably shorter than three months, and more preferably shorter than 30 day at -50 to 300 °C, because the culture is carried out using only an amount of the culture medium retained by the microporous body.

In addition, the present invention provides a method of culturing an organism over a relatively long period using the aforementioned organism-culture apparatus. In this method, the desired organism is adhered to the microporous body and is cultured under a suitable condition according to the same manner as that of the aforementioned examination method wherein a given amount of the culture medium is externally supplied to the microporous body at given intervals. An amount of the culture medium to be supplied can be calculated by measuring a weight reduction of the microporous body and the like.

Although, in principle, the culture in the aforementioned culture method of the present invention can be permanently carried out since a given amount of the culture medium is sequentially supplied, a culture period is usually shorter than 2 years, preferably shorter than 1 year, and more preferably shorter than 6 months at -50 to 300 °C depending on a subject organism.

### EXAMPLE

In order to make clear that organism cells can be cultured using the organism-culture apparatus of the present invention, experiments were performed using following apparatuses and samples.

### Culture experiment of plant tissue

### (1) Organism-culture apparatus

A cylindrical-type microporous body having an outer diameter of 2.1 cm, an inner diameter of 1.5 cm and a height of 6.5 cm which had been manufactured by firing at 1250 °C for 24 hours while containing 50% by weight of alumina (Al₂O₃) in Murakami clay (Niigata Prefecture in Japan)(manufactured by Kawasuzu Pottery & Co., lot no. CP0652115KS, water-absorbing ability 18.03% (wt/wt), porosity 36.80% (vol/vol), bulk density 2.041 (g/cm³)), was place in a 1L beaker, an opening of the beaker was sealed with an aluminum foil, and it was dry-sterilized at 161 °C for 2 hours. On the other hand, a dedifferentiation MS culture medium containing 2 ppm naphthalene acetic acid (NAA) and 2 ppm benzyladenine (BA) was sterilized in an autoclave, and the sterilized microporous body as described above was immersed in an adequate amount of the culture media in a clean bench. After allowing the microporous body to retain the culture medium, the microporous body was removed from the culture medium, an extra culture medium attached to the surface was removed and, then, it was placed in a culture test tube which was dry-sterilized according to the same condition as described above to prepare an organism-culture apparatus-1.

A disc-type microporous body having a diameter of 8.0 cm and a thickness of 0.8 cm which had been manufactured by firing PCTG No.1 clay (Tono Gaishi Co., Ltd.) at 1300 °C for 48 hours (manufactured by Tono Gaishi Co., Ltd., Lot No.CD0088000TG (T1), retained water 8.95% (wt/wt), porosity 18.20% (vol/vol), bulk density 2.03 (g/cm³)) was allowed to retain the MS culture medium as described above, and placed to a petri dish to prepare an organism-culture apparatus-2.

### (2) Test material

A seedling of Arabidopsis (*Arabidopsis thaliana*) colombia, a fungus *(Penicillium chrysogenum)* and a bacterium *(Bacillus subtilis)* were used as a test material. The seedling of *Arabiropsis* (5 cm) was sterilized by washing with running water, immersing in 70% ethanol for a few seconds followed by 5% aqueous sodium hypochlorite solution for 10 minutes. A leaf piece thereof was used.

### (3) Culture

The leaf piece of *Arabidopsis* was placed on a top portion of the cylindrical-type organism-culture apparatus-1 under an aseptic condition. Then, the opening was sealed again and the leaf piece was cultured under continuous light (3000 lux) at 26 °C. In addition, similarly, a fungus was planted to a top portion of the cylindrical-type organism-culture apparatus-1 to culture under the same condition. In addition, the fungus was planted also to a center portion of the disc-type organism-culture apparatus-2 to culture under the same condition. In addition, a suspension of a bacterium *Bacillus subtilis* was applied on a center portion of the disc-type organism-culture apparatus-2 to culture under the same condition.

### (4) Result

### Arabidopsis thaliana colombia lead piece

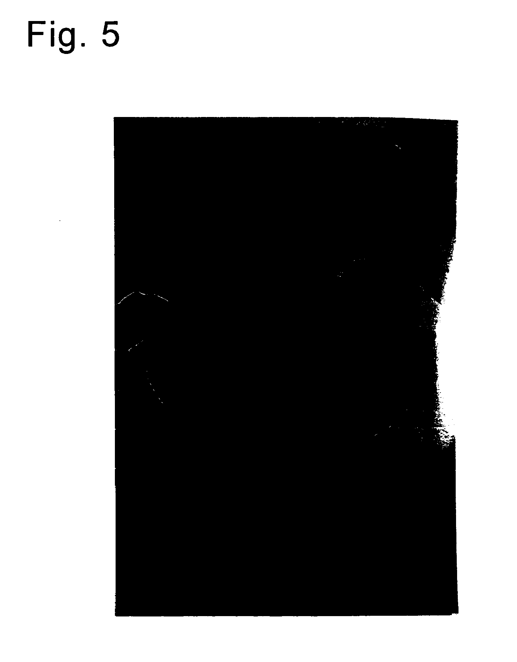

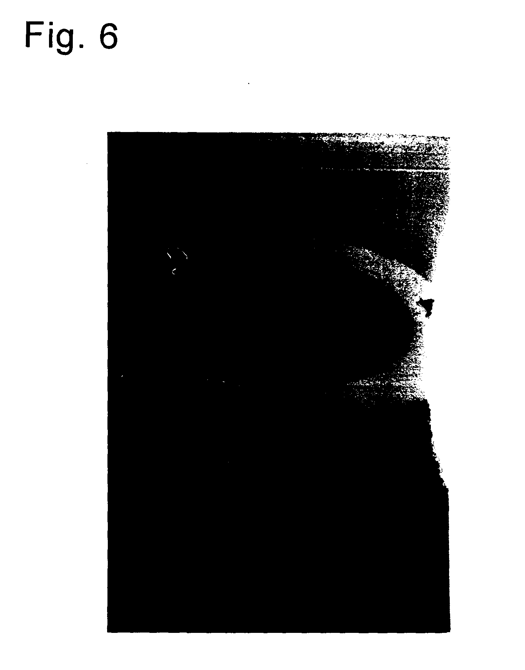

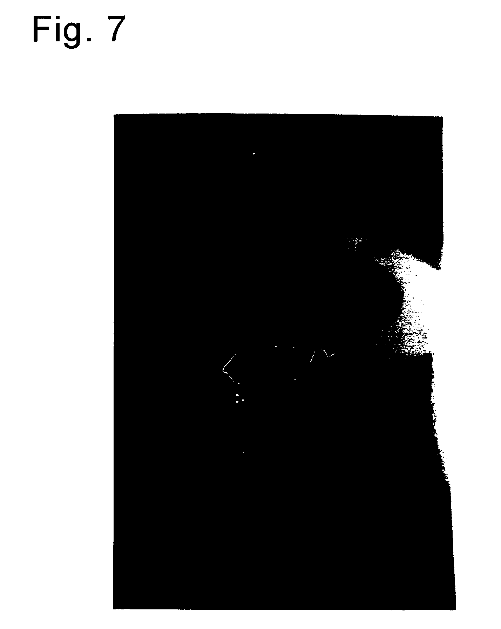

The results of a callus formation and a callus diameter of *Arabidopsis thaliana* placed on the organism-culture apparatus-1 as described above are shown in Tables 1 and 2. In addition, leaf piece states on 0, 10 and 21 days after starting the culture are illustrated on Figures 5-7, respectively.

**Table 1**

| Culture plant No. | 0 day | 10 day | 13 day | 21 day |
|---|---|---|---|---|
| 1 | - | + | + | + |
| 2 | - | - | - | + |
| 3 | - | - | + | + |
| 4 | - | - | - | + |
| 5 | - | + | + | + |
| 6 | - | - | - | + |
| 7 | - | - | - | + |
| 8 | - | - | - | + |

| | | | | |
|---|---|---|---|---|
| -:no callus formation +:callus formation | | | | |

**Table 2**

| Culture plant No. | Callus diameter (mm) | | | |
|---|---|---|---|---|
| | 0 day | 10 day | 13 day | 21 day |
| 1 | 0.0 | 0.9 | 1.2 | 1.6 |
| 2 | 0.0 | 0.0 | 0.0 | + |
| 3 | 0.0 | 0.0 | 0.8 | 1.5 |
| 4 | 0.0 | 0.0 | 0.0 | + |
| 5 | 0.0 | 1.3 | 1.8 | 2.2 |
| 6 | 0.0 | 0.0 | 0.0 | 1.2 |
| 7 | 0.0 | 0.0 | 0.0 | + |
| 8 | 0.0 | 0.0 | 0.0 | 0.7 |

| | | | | |
|---|---|---|---|---|
| +:callus formation, a diameter was not measured | | | | |

### Fungus (Penicillium chrysogenum) flora

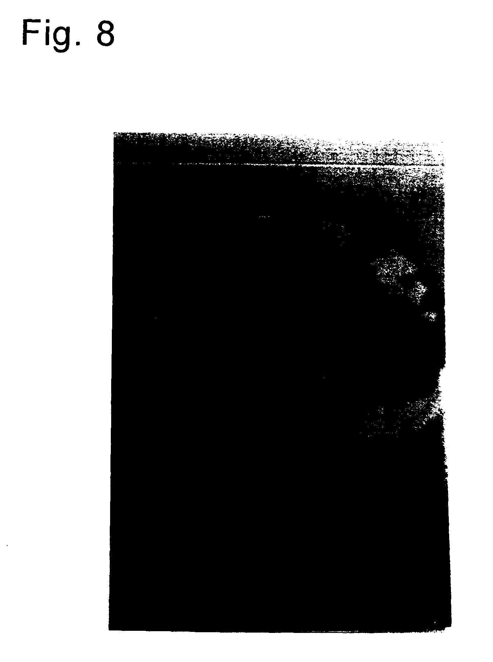

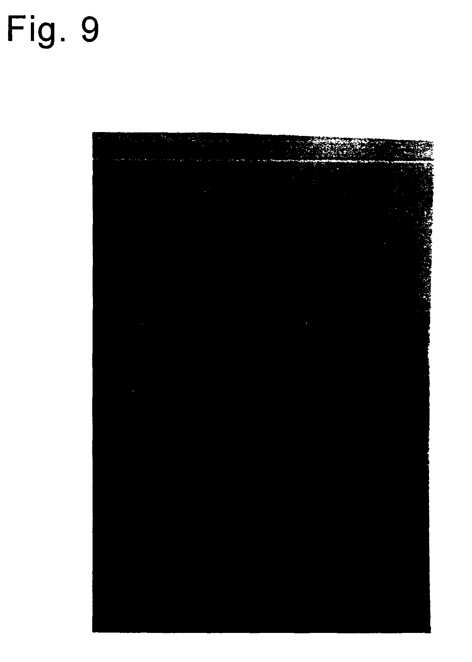

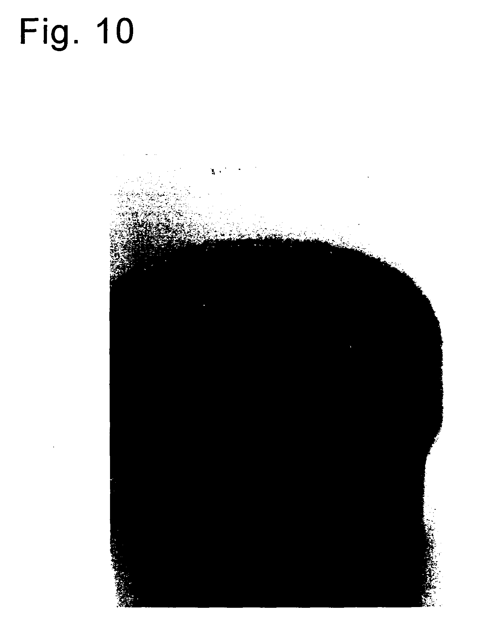

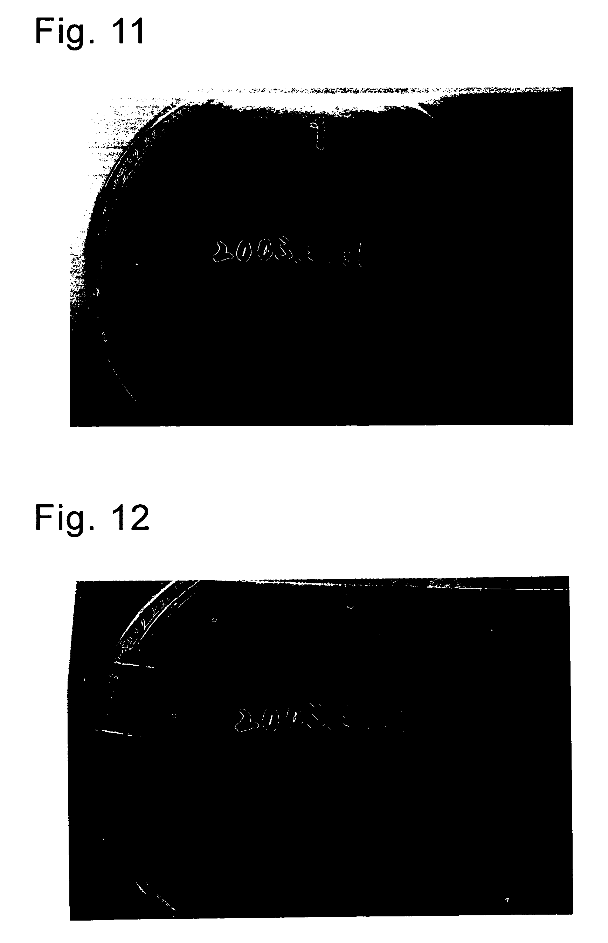

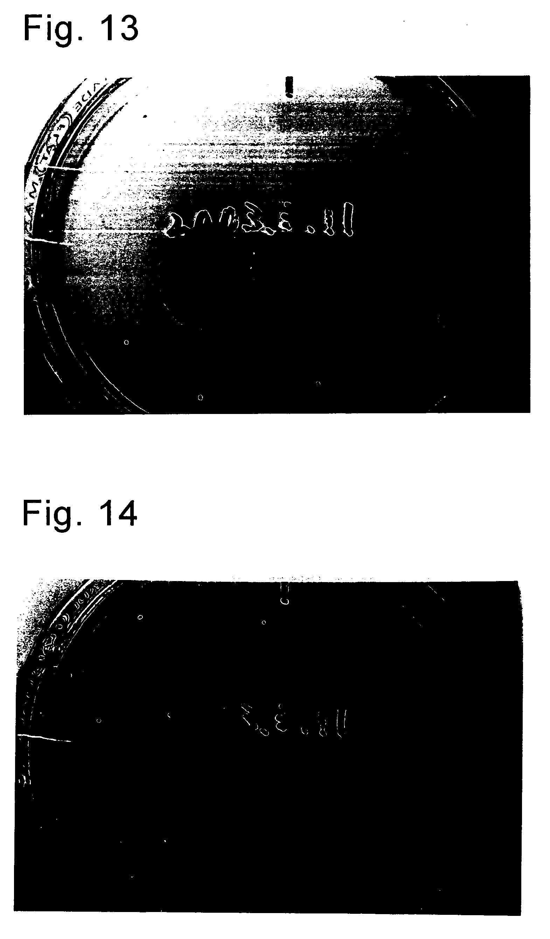

Transitional colony expansion of the fungus planted on the organism-culture apparatuses-1 and -2 as described above is shown in Tables 3 and 4. In addition, fungus states on 4, 10 and 13 days after starting the culture on the disc-type organism-culture apparatus-1 are illustrated on Figures 8-10, respectively. In addition, fungus states on 0, 13, 21 and 24 after starting the culture on the disc-type organism-culture apparatus-2 are illustrated on Figures11-14, respectively.

**Table 3**

| Culture fungus No. | Flora size (mm) | | | |
|---|---|---|---|---|
| | 0 day | 4 day | 10 day | 13 day |
| 1 | 0.0 | 4.2 | 12.4 | 13.8 |
| 2 | 0.0 | 1.6 | 3.6 | 6.9 |

**Table 4**

| Culture fungus No. | Flora size (mm) | | | |
|---|---|---|---|---|
| | 0 day | 13 day | 21 day | 24 day |
| 3 | 0.0 | 7.7 | 22.4 | 27.3 |

### Bacterium (Bacillus subtilis) flora

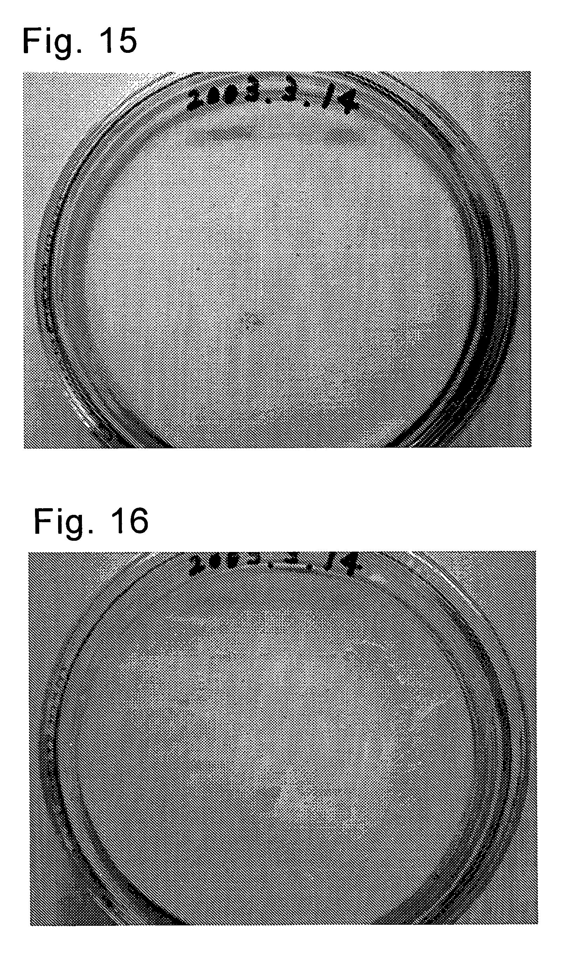

Transitional colony expansion of the bacteria planted on the organism-culture apparatus-2 as described above is shown in Table 5. In addition, bacterial states on 6 and 13 days after starting the culture on the disc-type organism-culture apparatus-2 are illustrated on Figures 15 and 16, respectively.

**Table 5**

| Cultured bacteria No. | 0 day | 1 day | 2 day | 4 day | 6 day | 13 day |
|---|---|---|---|---|---|---|
| 1 | - | - | - | + | + | + |

| | | | | | | |
|---|---|---|---|---|---|---|
| -: Flora was not visually observed +: Flora was visually observed | | | | | | |

In light of these results, it was confirmed that, in fact, plants can be dedifferentiated, and the fungus and bacteria can be propagated and the flora thereof can be visually observed using the organism-culture apparatus of the present invention, without being restricted a shape thereof.

### INDUSTRICALLY APPLICABLE FIELD

There can be provided an organism-culture apparatus which can be easily prepared and can be stored for a long period. The organism-culture apparatus can be recycled after sterilization and washing, and a use thereof is not restricted by a kind of a culture medium to be used, a pH, and a kind of an organism to be cultured. In addition, the number of micro-organisms can be exactly examined by counting the number of micro-organism colony on the surface of the organism-culture apparatus. In addition, a cultured plant can be removed from the microporous body in an intact state. Furthermore, even in the micro-organism examination in the space station, the micro-organism can be suitably cultured or grown in a high safety fashion, and such that an oxygen consumption and an increased weight accompanying an increased cost are suppressed.

## Claims

1. An organism-culture apparatus, which comprises a microporous body retaining a culture medium, wherein an organism is cultured on a surface of the microporous body.

2. The apparatus according to claim 1, wherein the microporous body retains 5-300% (wt/wt) of the culture medium based on the microporous body.

3. The apparatus according to claim 1 or 2, wherein the microporous body retains an amount of the culture medium retained by the capillary action thereof

4. The apparatus according to any one of claims 1-3, which does not comprise soil.

5. The apparatus according to any one of claims 1-4, wherein a cavity diameter in the microporous body is 5 µm or smaller.

6. The apparatus according to any one of claims 1-5, wherein a porosity of the microporous body is 10-80% (vol/vol).

7. The apparatus according to any one of claims 1-6, wherein the microporous body is a fired product of a non-metal inorganic solid material and retains 5-50% (wt/wt) of the culture medium.

8. The apparatus according to any one of claims 1-7, wherein the microporous body is a fired product of a non-metal inorganic solid material and the porosity of the microporous body is 10-50% (vol/vol).

9. The apparatus according to any one of claims 1-6, wherein the microporous body is an open-cell type plastic foam and retains 10-300% (wt/wt) of the culture medium.

10. The apparatus according to any one of claims 1-6 or 9, wherein the microporous body is an open-cell type plastic foam and the porosity of the microporous body is 10-80% (vol/vol).

11. The apparatus according to any one of claims 1-10, which is sealed in a sterile condition.

12. The apparatus according to any one of claims 1-11, wherein an organism to be cultured is a micro-organism.

13. The apparatus according to claim 12, wherein the micro-organism is bacteria, yeast, or fungi.

14. The apparatus according to claim 13, which is for a food examination.

15. The apparatus according to any one of claims 1-11, wherein the organism to be cultured is plants.

16. An organism-culture apparatus comprising a microporous body, wherein water in a culture medium retained in the microporous body has been substantially removed by drying, and wherein an organism is cultured on a surface of the microporous body to which an amount of water which has been removed is added before use.

17. An organism-culture apparatus, which comprises one or more of microporous bodies retaining a culture medium and a holding means for sealably holding the microporous bodies, wherein an organism is cultured on a surface of the microporous body.

18. A method of examining a micro-organism, which comprises steps of:
(1) sterilizing a microporous body retaining a culture medium by the capillary action thereof, or allowing a pre-sterilized microporous body to retain a pre-sterilized culture medium under a sterile condition;
(2) contacting a sample with the microporous body; and
(3) counting colonies formed on a surface of the microporous body after a culture of the microporous body under a given condition for a given period.

19. The method according to claim 18, wherein the culture in the step (3) is carried out at -50 to 300 °C within 6 months.

20. The method according to claim 18 or 19, wherein the micro-organism is bacteria, yeast, or fungi.

21. The method of examining according to any one of claims 18-20, wherein the micro-organism present in a food is examined.

22. A method of culturing an organism, which comprises steps of
(1) sterilizing a microporous body retaining a culture medium by the capillary action thereof, or allowing a pre-sterilized microporous body to retain a pre-sterilized culture medium under a sterile condition;
(2) adhering the organism to a surface of the microporous body; and
(3) after a culture of the microporous body under a given condition for a given period, successively supplying the microporous body with the culture medium such that an amount of the culture medium retained by the microporous body becomes an amount of the culture medium retained by the capillary action of the microporous body.

23. The method according to claim 22, wherein an organism to be cultured is a micro-organism.

24. The method according to claim 22 or 23, wherein the micro-organism is bacteria, yeast, or fungi.

25. The method according to claim 22, wherein the organism is plants.
